# EUROPEAN PATENT SPECIFICATION

(11) **EP 3 634 125 B1**
(45) Date of publication and mention of the grant of the patent: **29.12.2021**
(21) Application number: 18728168.8
(22) Date of filing: 05.06.2018
(51) Int. Cl.: A01N 1/02

(54) **SYSTEM AND METHOD FOR THE CONTROLLED TRANSPORT OF BIOLOGICAL SAMPLES**
SYSTEM UND VERFAHREN ZUM GESTEUERTEN TRANSPORT BIOLOGISCHER PROBEN
SYSTÈME ET PROCÉDÉ DE TRANSPORT RÉGULÉ D'ÉCHANTILLONS BIOLOGIQUES

(30) Priority: 06.06.2017 EP 17382339
(43) Date of publication of application: 15.04.2020
(73) Proprietor: General Courier Vallès, SL, 08403 Granollers (ES)
(72) Inventor: SÁNCHEZ GIRONELL, Marcel, 08188 Vallromanes (ES); SÁNCHEZ FERNÁNDEZ, Ariadna, 08188 Vallromanes (ES)
(74) Representative: Juncosa Miró, Jaime
(86) International application number: PCT/EP2018/064766
(87) International publication number: WO 2018/224496

(56) References cited:
- EP-A1- 2 668 847
- WO-A1-99/35453
- WO-A1-2015/112573
- US-A1- 2007 028 642
- US-A1- 2013 106 607
- US-A1- 2014 180 953

## Description

### Technical field

The present invention generally relates to systems for transporting biological samples. Particularly, the invention relates to a system and a method for the controlled transport of biological samples, for example vaccines or blood, as well as drugs needing a special preservation during transport, preferably to a medical/health center.

### Background of the Invention

Utility model CN-U-203788986 discloses an insulated transportable cooler for biological samples including a battery, a refrigeration device, an electronic control device, a screen, a door with an electrically controlled lock and a temperature sensor, in addition to wheels and a handle.

Utility model CN-U-202728908 discloses another insulated transportable cooler for biological samples including a battery, an electronic refrigerating sheet, an electronic control device, a screen and a temperature and humidity sensor. It also proposes to include a real time information transmission system.

Patent EP-B1-2341884 discloses a leak-tight bag for biological samples provided with a power supply source, a temperature sensor, a clock, and a system for downloading information, which allows controlling transport conditions and time.

Patent EP-B1-2190750 discloses a container for the metered dispensing of medication including a plurality of compartments intended for containing doses of medication, each provided with a sensor which allows detecting the presence of content therein, the container including an electronic control device, a battery, a screen, and a system for downloading information.

Patent application EP-A1-2436361 discloses a container for transporting biological samples or medical products provided with a cover integrating an electronic control device, a screen, control buttons, a connector for downloading information, sensors, a battery, and a motor driving a cam which causes the cover to be locked or released with respect to the container. The mentioned sensor can be a temperature sensor. Likewise, the authorized users can open or close the container by means of introducing a code through the control buttons, said code being able to be obtained by means of the mentioned information download.

US 2014180953 A1 discloses a method and system for controlled distribution of perishable goods including a remote central control station, a plurality of local control units and a plurality of mobile storage containers. Controlled conditions within the mobile storage containers are assured by wirelessly transmitting information between the emote central control system and local control units. The local control unit may measure e.g. the temperature inside the container, the location and speed of the container using GPS, sense acceleration, shocks and vibrations, and detect when the doors of the container are open.

EP2668847 A1 refers to a container for carrying biological samples comprising a location detection device and an inertial sensor for the position of the container and a control unit which comprises a communication device which is adapted to transmit the data that arrive from the location detection device and from the inertial position sensor to a central unit.

US20130106607 discloses a portable RDID tagged carrier for sterile implants and biological products, for supporting movement in product tracking and monitoring of regulated products such as tissue and biologics.

There is however the need for new systems and methods for the controlled transport of biological samples, for example blood, vaccines, infectious substances, among others, by means of a container which allow knowing at all times during transport the conditions of the biological samples and which in turn allow control/supervision of the monitored data of the container, assuring that any handling of the content of the container is recorded.

### Description of the Invention

In a first aspect, the present invention provides a system for the controlled transport of biological samples as defined in claim 1. The system comprises like in the state of the art a container including a main body defining a housing cavity; a cover; an opening/closure control device for controlling the opening/closure of the container; a temperature sensor (more than one can be included) configured for providing information about the internal temperature of the container from an instant of loading the biological samples, for example blood or vaccines, among others, in the container to a subsequent instant of opening the container and/or extracting the biological samples from the container; and an electronic board configured for collecting data acquired by the mentioned temperature sensor and for controlling the state and the operation of the opening/closure control device.

The proposed system further comprises a server with one or more processors and at least one database, or memory. The server is preferably located in a remote area with respect to the container, so the communication module sends the data to the server by means of a wireless connection. The server can be a physical server, a local server, or a cloud server.

Likewise, the container includes a biological sample content detector (more than one can be included) which is controlled by the mentioned electronic board. The electronic board further includes a communication module, such as a GSM module or another radiotechnology module, configured for sending at least a series of data about the temperature (of the inside of the container) and the content of the container, during transport, to the server for storage of the data in the database, or memory.

The system also preferably includes a communication device such as a mobile telephone or a tablet that is located, in operative situation, in the proximity of the container, and comprising positioning means (for example, a GPS positioning module) operatively connected to the server to allow tracking positioning data of the container during transport.

The biological sample content detector is a proximity sensor selected from a capacitive, inductive or infrared sensor, configured for detecting the presence of the biological samples transported inside the container from the instant of loading the biological samples in to the instant of extracting the biological samples from the container.

It is particularly important for the proposed system to assure detection of this extraction of the biological samples, extracting only a part of the biological samples from the container thereby being prevented.

The opening/closure control device is preferably included in the cover of the container.

The opening/closure control device comprises an electromechanical device configured, together with the mentioned electronic board, for being activated by means of two RFID sensors depending on the type of biological samples to be transported.

By means of using the two RFID sensors, the user who opens/closes/handles the container is detected. Likewise, the two RFID sensors allow detecting the name of the center of origin and destination of the biological samples.

The container further comprises one or more batteries for self-powering of the electronic board.

The container also preferably includes a display unit. The display unit can comprise a screen, for example a TFT screen, and/or light-emitting indicators, for examples LEDs. If the display unit is a screen, in addition to showing discrete information about the content related with the transported biological samples, i.e., whether the container is full or empty, it can also show information relating to the state of the battery/batteries, the temperature inside the container, the user/operator transporting the container, the type of transported sample, the center where the samples were collected, the center where the samples will be transported, etc. If the display unit is LEDs, information about the content of the container can be shown by means of LEDs of different colors.

Likewise, the container can comprise an accelerometer configured for detecting sudden movements of the container during transport or handling.

In another aspect, the present invention provides a method for the controlled transport of biological samples in a container, as defined in claim 10. The method comprises the following steps:
reading by a RFID reader data from at least two different RFID tags at a given configured period of time, wherein a first RFID tag includes data of a center of origin and a second RFID tag includes data of a center of destination of the container as well as the type of biological sample to be transported;
detecting, by means of a content detector controlled by an electronic board of the container, the existence of a biological sample load in the container; sending to a server, by means of a communication module included in the electronic board, a series of data about the internal temperature of the container, the content and incidences of opening/closing the container and/or incidences related with content handling; and storing said series of received data and time instants of said incidences of opening/closing the container in a database of the server, wherein the data stored in the database constitutes a control and/or supervision record.

In one embodiment, the method comprises emitting a warning signal if the content detector detects content inside the container once the biological samples have been extracted therefrom.

In another embodiment, the method comprises emitting a warning signal if the temperature of the container is the same as or exceeds a pre-established threshold temperature value during transport.

In yet another embodiment, the method tracks positioning data of the container from an instant of closing to an instant of opening the container, during transport, using positioning means of a computing device (for example the mobile telephone of the operator transporting the container) operatively connected to the server and located in the proximity of the container.

### Brief Description of the Drawings

The foregoing and other advantages and features will be more clearly understood based on the following detailed description of several illustrative and non-limiting embodiments in reference to the attached drawings, in which:
Fig. 1 schematically shows an embodiment of a system for the controlled transport of biological samples with detail of the scenario and basic intervening agents.
Fig. 2a to 2d show different views of an embodiment of the container for transporting biological samples.
Figs. 3a and 3b illustrate a detail of the elements constituting the closing device of the container.
Figs. 4a and 4b show a possible embodiment of a secondary container for the biological samples, vaccines or drugs, used in the system.
Fig. 5a and 5b are two schematic views illustrating the detection of the secondary container inside the main container, with a proximity detector.
Fig. 6 shows an embodiment of the display unit of the container of Fig. 2.
Fig. 7 is a flow chart describing in detail an embodiment of a method for the controlled transport of biological samples in a container according to this invention.

### Detailed Description of several Embodiments

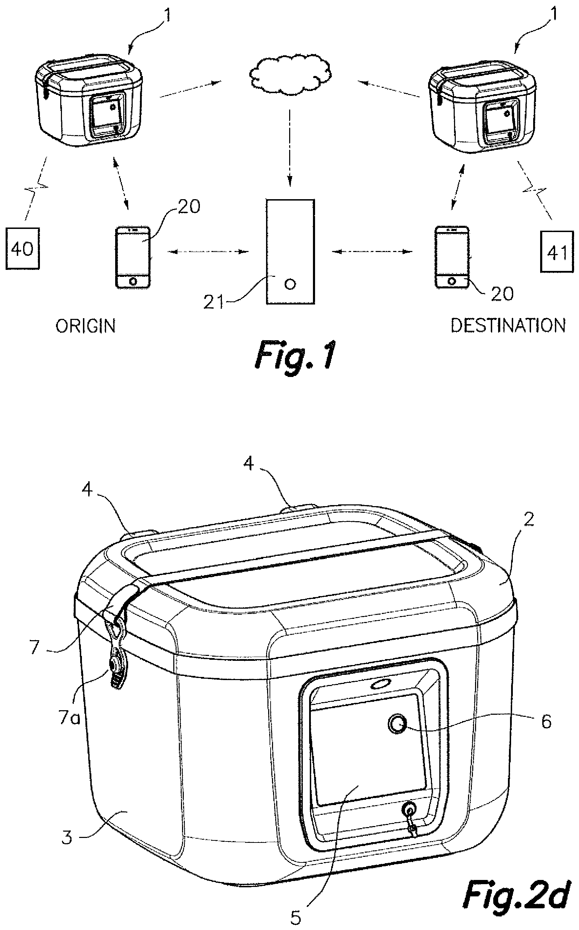

Fig. 1 illustrates an embodiment of a system for the controlled transport of biological samples. According to this embodiment, the system comprises a container 1 for storing and transporting biological samples, for example blood samples, vaccines, or drugs among others, including a communication module; a server 21 operatively connected to the mentioned container 1 through a communications network such as the Internet; a computing device 20 such as a mobile telephone, preferably belonging to the operator (not illustrated for simplicity of the drawing) in charge of transporting the biological samples, and at least two RFID tags 40, 41. The positioning means, for example GPS, integrated in the computing device 20 itself can therefore be used to allow tracking the positioning data of the container 1 in the transportation route.

The system shown in the figure includes two different RFID tags 40, 41, and the container 1 further includes a RFID reader that is configured to read data of said different RFID tags 40, 41 at a given configured period of time, wherein a first RFID tag 40 includes data of a center of origin and a second RFID tag 41 includes data of a center of destination of the container 1 as well as type of biological sample to be transported.

The mobile computing device 20 with wireless communication with the communication module of the container 1, via a mobile application installed therein, is further used at the origin to validate the data and center of origin associated to the container 1 and at destination to validate the data and center of destination and to activate the RFID reader, the system further including a warning unit to emit an alarm in case of error. On this way the electronic components installed in the container 1 can be in stand-by state during transport of the container except during the periods in which they transmit data to the server 21, and are woken up at destination just before authorized personnel approach the container 1 with a RFID tag 41 to open the container 1.

Alternatively, the server 21 could be a cloud server.

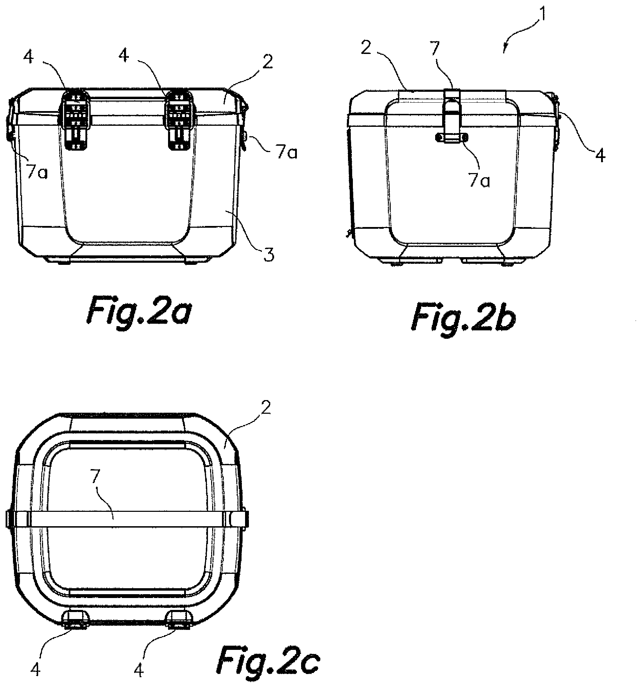

Fig. 2a to 2d illustrates an embodiment of the container 1 proposed by the present invention. The container 1 consists of a rigid outer body (or main body) 3 manufactured in plastic materials with an inner foam body (preferably 4 cm thick) conferring it with insulating capacity for keeping the cold. The container 1 has an upper cover 2 fixed with hinges 4. The cover 2 is assembled to the main body 3 of the container 1, generating a leak-tight inner space. To assure leak-tightness, there is a plastic profile both in the upper part of the main body 3 and in the lower part of the cover 2 fitting with one another and incorporating a rubber profile which, when the cover 2 is closed, act as a sealing gasket and make it leak-tight. The container 1 can also include metal hooks on the sides of the main body 3 for holding a strap making it easier to transport the container 1. Likewise, the main body 3 can include on one of its inner sides an elastic band held by rivets fixed at each end which allows holding a folder containing information about the biological samples transported in the container 1. The container further includes a handle portion 7 (in the example an adjusting strap) and fixing elements 7a at both ends of the handle.

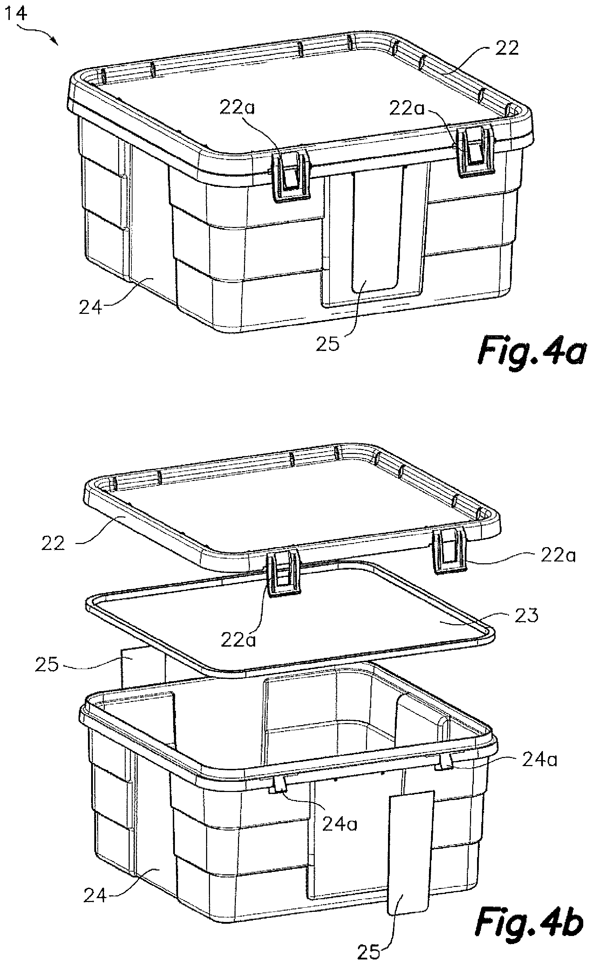

The inside of the container 1 is lined with a white rigid body of the same material as the outside thereof in order to make it leak-tight and to convert it into a leak-tight space. Preferably, the inner area is sized for storing four racks of biological samples housed inside a secondary container 14 (see Figs. 4a and 4b for a possible embodiment, and 5a and 5b showing the arrangement of secondary container 14 inside main container 1) manufactured preferably in a plastic, transparent material, with an opaque cover, in addition to another secondary container having the same or less height for storing different biological samples. The inner area also includes a temperature sensor (not illustrated). The temperature sensor, more than one can be included, provides information about the internal temperature of the container 1 from an instant of loading the biological samples in the container 1 to the instant of opening the container 1 and/or extracting the biological samples from the container 1. The inner area can also include a humidity sensor for providing information about the humidity inside the container 1.

The container 1 also includes an electronic board and a content detector (also not illustrated). The electronic board incorporates the mentioned communication module, for example a GSM or UMTS module, for establishing the mentioned communication with the server 21, sending to said server 21 at least the data about the temperature acquired by the temperature sensor and by the content detector for the storage thereof in its database, or memory.

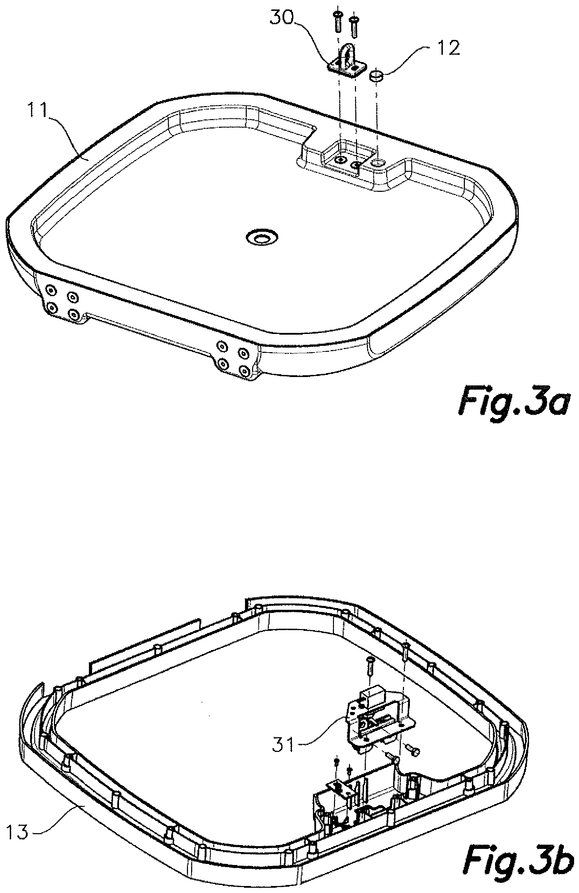

Likewise, the mentioned cover 2 of the container 1 includes, partly illustrated in Figs. 3a and 3b, an opening/closure control device for controlling the opening/closure of the container 1. The opening/closure control device comprises an electromechanical device 31 configured, together with the mentioned electronic board, for being activated by means of at least two RFID sensors 40, 41 depending on the type of biological samples to be transported and location (origin or destination). In other words, if the biological samples to be transported are blood samples a first RFID 40, 41 sensors will be used and if the biological samples to be transported are vaccines a second RFID 40, 41 sensors will be used. The cover 2 can also include hooks holding a net which allows holding a series of cold packs, depending on the needs of each case. Fig. 3a also includes a detail of a magnet 12 to be use in conjunction with a Hall detector for the closure. A latch 30 is also indicated cooperating with another latch in the cover 2. The frame 1 1 of Fig. 3a is arranged within a housing of frame 13.

The electronic board comprises one or more microprocessors for operating/controlling the different peripherals/elements of the container 1: display unit 5, RFID identification system for identifying the type of RFID tag or cards 40, 41 indicating origin, destination and biological samples and the users/operators, communication module, content detector, opening/closure control device, temperature sensor, as well as for accommodating supply voltages for the different peripherals/elements, etc.

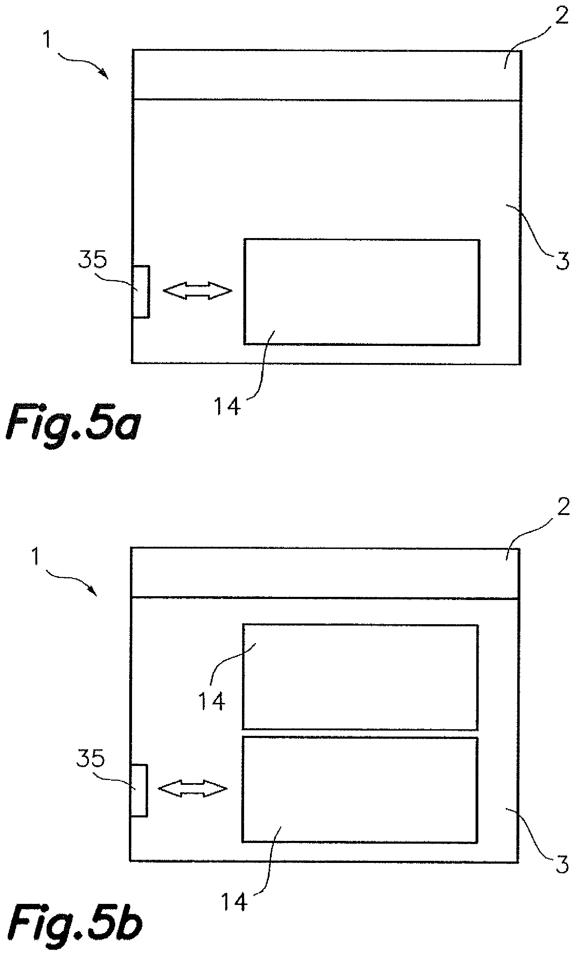

The content detector is preferably a photoelectric proximity sensor 35 that works by means of infrared. Alternatively, capacitive or inductive proximity sensors can likewise be used for said functionality. Figs. 5a and 5b show how the secondary container 14 is detected inside the main container 1, by means of a proximity detector 35. The secondary container as seen in Figs. 4a and 4b, will be made of a transparent or translucid material but having a plate or label 25 that allow a proximity sensor such as an infrared sensor to be operative.

The container 1 further includes one or more batteries for self-powering of the electronic board.

In the embodiment described in Figs. 2a to 2d, the display unit 5 comprises a screen, for example a TFT screen. The display unit can be tilted to thereby allow viewing same from different angles. The display unit 5 incorporates a push-button 6 activating the light of the screen 5 at the user's/operator's demand for consulting the data appearing thereon.

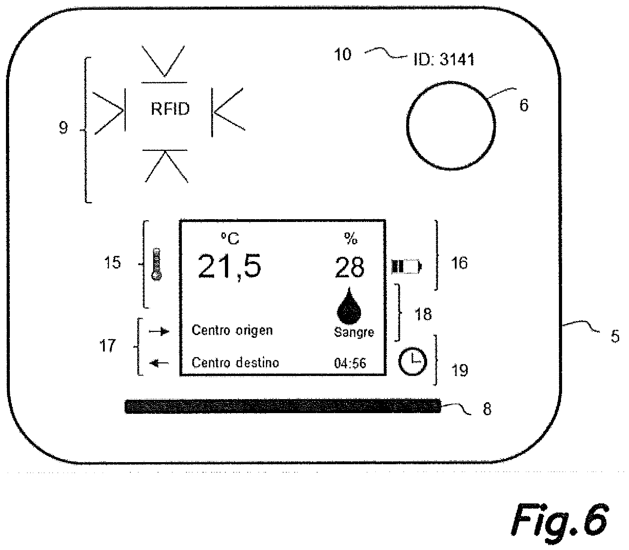

Fig. 6shows an embodiment of the mentioned screen. As can be seen in Fig. 6, the screen shows data 15 relating to the internal temperature of the container 1, to the remaining percentage of battery 16, to the medical/health center of origin of the biological samples and the center of destination of the biological samples 17, to the type of samples 18 (in this particular case, blood samples, as seen in the text and in the symbol of a drop of blood, it must be pointed out that for other types of samples other symbols will be used (for example a cross to indicate vaccines)) and to the time elapsed during transport 19 (from the closure of the container to the opening thereof in the center of destination). The upper area of the screen includes the reading area of the RFID sensors 9, the identification number 10 of the container 1 and the mentioned push-button 6. The lower area of the screen includes a LED 8 which changes color depending on whether the container is full or empty (preferably, red if the container 1 is full and green if it is empty).

The mentioned screen 5 can also be automatically activated, i.e., the screen 5 can be configured to light up for specific time period.

In an alternative embodiment, the container 1 can include two optoelectronic components, such as LEDs of different colors, to distinctly indicate whether the container 1 is full or empty.

Optionally, the container 1 can include an accelerometer for detecting sudden movements of the container 1 during transport or handling.

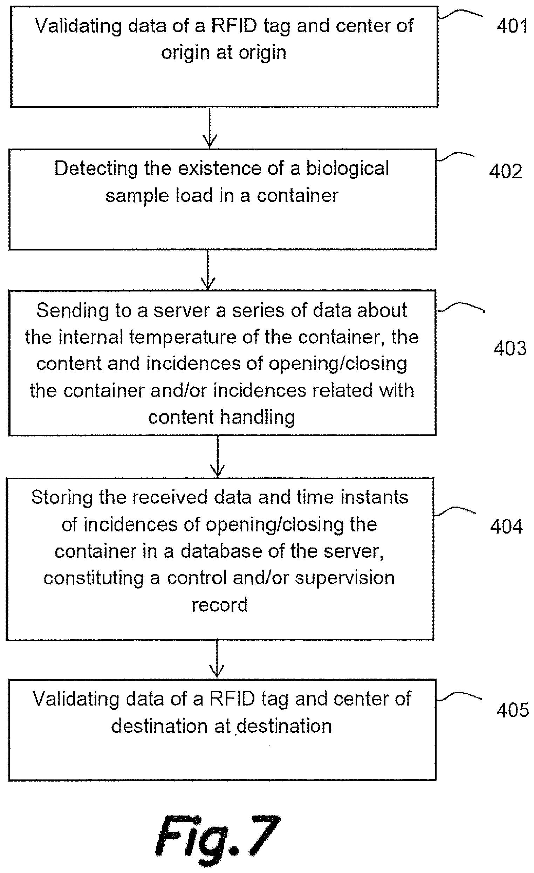

Now, in reference to Fig. 7, said figure shows an embodiment of a method for the controlled transport of biological samples in a container, such as the one described above. The method comprises first validating data of RFID tag 40 and center of origin at origin, S401 and detecting, step 402, by means of the mentioned content detector, the existence of a biological sample load in the secondary container 14 within the container 1. Then, the method comprises sending to the server 21, step 403, by means of the mentioned communication module included in the electronic board of the container 1, every certain configurable period of time, a series of data at least relating to: the internal temperature of the container 1, the content and incidences of opening/closing the container 1. Finally, the method stores, step 404, the series of received data and the time instants of said incidences of opening/closing the container 1 in a database of the server 21, wherein the data stored in the database constitutes a control and/or supervision record. Finally, on step 405 data of RFID tag 40 and center of destination are validated.

Likewise, the mentioned communication module can also send information relating to the state of the battery/batteries, the medical/health center of origin of the biological samples, the route taken, the identifier of the container 1, the user/operator identifier, in addition to other information memorized in the RFID sensors that may be personalized as required, for example, incidences occurring during transport, such as the wait time, if the container 1 has broken, if an accident has occurred in the route taken, the traffic condition in the route taken, etc.

In one embodiment, the method emits a warning signal if the content detector detects content inside the container 1 once the biological samples have been extracted therefrom. It is thereby assured that all the transported biological samples are extracted in the medical/health center of destination.

Furthermore, if the internal temperature of the container 1 is the same as or exceeds a pre-established threshold temperature value during transport, for example 23 degrees for blood samples or 8 degrees for vaccines, a warning signal can be emitted, said emitted warning signal being recorded in the server 21.

The method allows tracking positioning data of the container 1 from the instant of closing to the instant of opening same, during transport. The mentioned positioning means of the computing device 20 are preferably used for such purpose. Nevertheless, a positioning sensor could be alternatively included in the container 1. The container 1 itself, without having to use the computing device 20, will thereby allow tracking the positioning data of the container 1 during transport.

As improvements of the present invention, the present invention can also provide a mobile application, or an APP, run in the computing device 20 or in another computing device, providing through a user interface the same information as the data stored in the mentioned database (internal temperature of the container, content, incidences, state of the battery/batteries, etc.).

By means of cited APP running in the computing device 20 it is also possible that one or several containers being automatically open when arriving to the destination, as detected by the computing device 20 of the carrier at that place.

Secondary container 14 illustrated in Figs. 4a and 4b, comprises a body 24 which integrates in a side wall a plate 25, and further comprising a lid 22 with a watertight element 23. The lid further including fixing appendixes 22a to be coupled to protrusions 24a of the body 24. Likewise, the container 1 complies with UN 3373 guidelines in force on the date of filing of this document.

The scope of the present invention is defined in the attached claims.

## Claims

1. A system for the controlled transport of biological samples, comprising:
- a server (21) including a database; and
- a container (1) for storing biological samples including:
- a main body (3) defining a housing cavity;
- a cover (2);
- an opening/closure control device for controlling the opening/closure of the container (1);
- at least one temperature sensor configured for providing information about the internal temperature of the container (1) from an instant of loading said biological samples in the container (1) to a subsequent instant of opening the container (1) and/or extracting the biological samples from the container (1); and
- an electronic board configured for collecting data acquired by said at least one temperature sensor, and for controlling a state and operation of said opening/closure control device, wherein the electronic board includes a communication module configured for sending to said server (21), during transport, a series of data for the storage of said series of data in the database of the server (21);
wherein:
- the system further includes two different RFID tags (40, 41), wherein a first RFID tag (40) includes data of a center of origin and a second RFID tag (41) includes data of a center of destination of the container (1) as well as the type of biological sample to be transported;
- the container (1) further includes a RFID reader that is configured to read data of said different RFID tags (40, 41) at a given configured period of time;
- the opening/closure control device comprises an electromechanical device which is configured for being activated, during opening or closure, using the RFID tags (40, 41); and
- said container (1) further includes a detector of biological samples within the container (1) controlled by said electronic board,
wherein said detector of biological samples is a proximity sensor selected from infrared, capacitive or inductive detector, the proximity sensor being configured for detecting the presence of the biological samples transported inside the container (1) from the instant of loading the biological samples in to the instant of extracting the biological samples from the container (1); and
wherein the series of data sent by the electronic board comprises data about the content of the container (1) acquired by the detector and data about the internal temperature of the container (1),
such that the system assures that any handling of the content of the container (1) is recorded.

2. The system according to claim 1, wherein said biological samples are held within a secondary container (14) arranged inside the container (1).

3. The system according to claim 2, wherein the secondary container (14) is made of a transparent or translucid material and further comprises a label, plate or texturized zone (25) to cooperate with the proximity sensor.

4. The system according to any one of the preceding claims, wherein the opening/closure control device is included in the cover (2).

5. The system according to claim 1, further comprising a mobile computing device (20) configured for wireless communication with said communication module and with the RFID reader, via a mobile application installed therein, wherein said mobile computing device (20) is configured for being used at the origin to validate the data and center of origin associated to the container (1) and at the destination to validate the data and center of destination and to activate the RFID reader, the system further including a warning unit to emit an alarm in case of error.

6. The system according to any one of the preceding claims, further comprising a display unit (5) controlled by the electronic board for indicating distinct information about the content of the container (1) that is at least related with the transported biological samples wherein said display unit comprising a screen and/or light-emitting indicators.

7. The system according to any one of the preceding claims, wherein the container (1) further comprises one or more batteries for self-powering of the electronic board.

8. The system according to any one of the preceding claims, wherein the container (1) further comprises at least one accelerometer configured for detecting sudden movements of the container (1) during transport or handling.

9. The system according to any one of the preceding claims, further comprising a computing device (20), including a mobile telephone or a tablet, located in the proximity of the container (1), during transport, said computing device (20) comprising positioning means operatively connected to the server (21), to allow tracking positioning data of the container (1) during transport.

10. A method for the controlled transport of biological samples in a container, wherein said container (1) comprises:
- a main body (3) defining a housing cavity;
- a cover (2);
- a RFID reader;
- an opening/closure control device for controlling the opening/closure of the container (1), wherein the opening/closure control device comprises an electromechanical device configured for being activated, during opening or closure, using at least two different RFID tags (40, 41);
- at least one temperature sensor providing information about the internal temperature of the container (1) from an instant of loading the biological samples in the container (1) to a subsequent instant of opening the container (1) and/or extracting the biological samples from the container (1); and
an electronic board configured for collecting the data acquired by at least said temperature sensor and for controlling a state and operation of said opening/closure control device;
the method comprising the following steps:
reading by the RFID reader data from the at least two different RFID tags (40, 41) at a given configured period of time, wherein a first RFID tag (40) includes data of a center of origin and a second RFID tag (41) includes data of a center of destination of the container (1) as well as the type of biological sample to be transported;
detecting, by means of a detector of biological samples controlled by said electronic board, the existence of a biological sample load in the container (1);
wherein said detector of biological samples is a proximity sensor selected from infrared, capacitive or inductive detector, the proximity sensor being configured for detecting the presence of the biological samples transported inside the container (1) from the instant of loading the biological samples in to the instant of extracting the biological samples from the container (1),
sending to a server (21), by means of a communication module included in the electronic board, a series of data about the internal temperature of the container (1), the content and incidences of opening/closing the container (1) and/or incidences related with content handling; and
storing said series of received data and time instants of said incidences of opening/closing the container (1) in a database of the server (21),
wherein said data stored in said database constitutes a control and/or supervision record.

11. The method according to claim 10, further comprising emitting a warning signal if the content detector continues to detect content after extracting the biological samples from the container (1) and/or emitting a warning signal if the temperature of the container (1) is the same as or exceeds a pre-established threshold temperature value during transport.

12. The method according to claims 10 or 11, wherein said sending of the series of data is performed every certain configurable period of time.

13. The method according to claim 10, further comprising tracking positioning data of the container (1) from an instant of closing the container (1) to an instant of opening the container (1), during transport, using positioning means of a mobile computing device (20) operatively connected to the server (21), said mobile computing device (20) further being used by means of a mobile application installed therein for validating at an origin the data of RFID tag (40) and center of origin associated to the container (1) and at a destination to validate the data of RFID tag (41) and center of destination of the container (1) and to activate the RFID reader, the system further including a warning unit to emit an alarm in case of error.

## Patentansprüche

1. System zum gesteuerten Transport biologischer Proben, umfassend:
- einen Server (21) mit einer Datenbank; und
- einen Behälter (1) zur Aufbewahrung biologischer Proben umfassend:
- einen Hauptkörper (3), der einen Aufnahmehohlraum bildet;
- einen Deckel (2);
- eine Öffnungs-/Schließsteuerungsvorrichtung zum Steuern des Öffnens/Schließens des Behälters (1);
- mindestens einen Temperatursensor, der zur Bereitstellung von Informationen über die Innentemperatur des Behälters (1) ab dem Zeitpunkt des Einbringens der genannten biologischen Proben in den Behälter (1) bis zum späteren Zeitpunkt des Öffnens des Behälters (1) und/oder der Entnahme der biologischen Proben aus dem Behälter (1) ausgelegt ist; und
- eine Elektronikkarte, die zum Sammeln der durch den genannten mindestens einen Temperatursensor erfassten Daten und zum Steuern des Zustands und der Arbeitsweise der genannten Öffnungs-/Schließsteuerungsvorrichtung ausgelegt ist, wobei die Elektronikkarte ein Kommunikationsmodul enthält, das so ausgelegt ist, dass es während des Transports eine Reihe von Daten an den genannten Server (21) zwecks Speicherung der genannten Reihe von Daten in der Datenbank des Servers (21) sendet;
wobei:
- das System ferner zwei verschiedene RFID-Transponder (40, 41) umfasst, wobei ein erster RFID-Transponder (40) Daten über einen Ausgangsort und ein zweiter RFID-Transponder (41) Daten über einen Zielort des Behälters (1) sowie die Art der zu transportierenden biologischen Probe enthält;
- der Behälter (1) ferner ein RFID-Lesegerät enthält, das zum Lesen von Daten der genannten verschiedenen RFID-Transponder (40, 41) in einer bestimmten festgelegten Zeitspanne ausgelegt ist;
- die Öffnungs-/Schließsteuerungsvorrichtung eine elektromechanische Vorrichtung umfasst, die so ausgelegt ist, dass sie mittels der RFID-Transponder (40, 41) während des Öffnens oder Schließens aktiviert wird; und
- der genannte Behälter (1) ferner einen von der genannten Elektronikkarte gesteuerten Detektor für biologische Proben innerhalb des Behälters (1) umfasst,
wobei der genannte Detektor für biologische Proben ein aus einem Infrarot-, kapazitiven oder induktiven Detektor ausgewählter Näherungssensor ist, wobei der Näherungssensor so ausgelegt ist, dass er das Vorhandensein der in dem Behälter (1) transportierten biologischen Proben ab dem Zeitpunkt des Einbringens der biologischen Proben in den Behälter bis zum Zeitpunkt der Entnahme der biologischen Proben aus dem Behälter (1) erfasst; und
wobei die von der Elektronikkarte gesendete Datenreihe Daten über den vom Detektor erfassten Inhalt des Behälters (1) und Daten über die Innentemperatur des Behälters (1) umfasst, sodass mit dem System sichergestellt wird, dass jedwede Handhabung des Inhalts des Behälters (1) aufgezeichnet wird.

2. System nach Anspruch 1, wobei die genannten biologischen Proben in einem im Inneren des Behälters (1) angeordneten Zweitbehälter (14) untergebracht sind.

3. System nach Anspruch 2, wobei der Zweitbehälter (14) aus einem transparenten oder lichtdurchlässigen Material besteht und ferner eine mit dem Näherungssensor zusammenwirkende Marke, Plakette oder texturierte Fläche (25) aufweist.

4. System nach einem der vorstehenden Ansprüche, wobei die Öffnungs-/ Schließsteuerungsvorrichtung in dem Deckel (2) enthalten ist.

5. System nach Anspruch 1, ferner umfassend eine mobile Rechnereinheit (20), die zur drahtlosen Kommunikation mit dem genannten Kommunikationsmodul und dem RFID-Lesegerät über eine darin integrierte mobile Anwendung ausgelegt ist, wobei die genannte mobile Rechnereinheit (20) so ausgelegt ist, dass sie am Ausgangsort zur Validierung der mit dem Behälter (1) verknüpften Ausgangsdaten bzw. des Ausgangsortes und am Zielort zur Validierung der Zieldaten bzw. des Zielortes sowie zur Aktivierung des RFID-Lesegerätes verwendet wird, wobei das System ferner eine Warneinheit zur Abgabe eines Alarms bei Auftreten eines Fehlers umfasst.

6. System nach einem der vorstehenden Ansprüche, ferner umfassend eine von der Elektronikkarte gesteuerte Display-Einheit (5) zur Anzeige bestimmter Informationen über den Inhalt des Behälters (1), die zumindest mit den transportierten biologischen Proben in Zusammenhang stehen, wobei die genannte Display-Einheit einen Bildschirm und/oder lichtemittierende Anzeigeelemente umfasst.

7. System nach einem der vorstehenden Ansprüche, wobei der Behälter (1) ferner eine oder mehrere Batterien zur Eigenstromversorgung der Elektronikkarte umfasst.

8. System nach einem der vorstehenden Ansprüche, wobei der Behälter (1) ferner mindestens einen Beschleunigungsmesser, der zur Erfassung plötzlicher Bewegungen des Behälters (1) während Transports oder Handhabung ausgelegt ist, umfasst.

9. System nach einem der vorstehenden Ansprüche, umfassend ferner eine während des Transports in der Nähe des Behälters (1) befindliche Rechnereinheit (20) in Form eines Mobiltelefons oder Tablets, wobei die genannte Rechnereinheit (20) eine mit dem Server (21) operativ verbundene Positionierungseinrichtung zur Verfolgung der Positionsdaten des Behälters (1) während des Transports umfasst.

10. Verfahren zum gesteuerten Transport biologischer Proben in einem Behälter, wobei der genannte Behälter (1) Folgendes umfasst:
- einen Hauptkörper (3), der einen Aufnahmehohlraum bildet;
- einen Deckel (2);
- ein RFID-Lesegerät;
- eine Öffnungs-/Schließsteuerungsvorrichtung zum Steuern des Öffnens/Schließens des Behälters (1), wobei die Öffnungs-/Schließsteuerungsvorrichtung eine elektromechanische Einheit umfasst, die so ausgelegt ist, dass sie beim Öffnen oder Schließen unter Einsatz mindestens zweier verschiedener RFID-Transponder (40, 41) aktiviert wird;
- mindestens einen Temperatursensor, der zur Bereitstellung von Informationen über die Innentemperatur des Behälters (1) ab dem Zeitpunkt des Einbringens der biologischen Proben in den Behälter (1) bis zum späteren Zeitpunkt des Öffnens des Behälters (1) und/oder der Entnahme der biologischen Proben aus dem Behälter (1) ausgelegt ist; und
- eine Elektronikkarte, die zum Sammeln der durch den mindestens einen genannten Temperatursensor erfassten Daten und zum Steuern des Zustands und der Arbeitsweise der genannten Öffnungs-/Schließsteuerungsvorrichtung ausgelegt ist;
wobei das Verfahren die folgenden Schritte umfasst:
Lesen der Daten aus den mindestens zwei verschiedenen RFID-Transpondern (40, 41) in einer bestimmten festgelegten Zeitspanne durch das RFID-Lesegerät, wobei ein erster RFID-Transponder (40) Daten über einen Ausgangsort und ein zweiter RFID-Transponder (41) Daten über einen Zielort des Behälters (1) sowie die Art der zu transportierenden biologischen Probe enthält;
Erfassen des Vorhandenseins einer Füllung biologischer Proben in dem Behälter (1) mittels eines von der genannten Elektronikkarte gesteuerten Detektors für biologische Proben;
wobei der genannte Detektor für biologische Proben ein aus einem Infrarot-, kapazitiven oder induktiven Detektor ausgewählter Näherungssensor ist, wobei der Näherungssensor so ausgelegt ist, dass er das Vorhandensein der in dem Behälter (1) transportierten biologischen Proben ab dem Zeitpunkt des Einbringens der biologischen Proben in den Behälter bis zum Zeitpunkt der Entnahme der biologischen Proben aus dem Behälter (1) erfasst,
Senden einer Reihe von Daten über Innentemperatur und Inhalt des Behälters (1) sowie die Vorgänge des Öffnens/Schließens des Behälters (1) und/oder im Zusammenhang mit der Handhabung des Inhalts an einen Server (21) mittels eines in der Elektronikkarte enthaltenen Kommunikationsmoduls; und
Speichern der genannten Reihe empfangener Daten und Zeitpunkte der genannten Vorgänge des Öffnens/Schließens des Behälters (1) in einer Datenbank des Servers (21),
wobei die in der genannten Datenbank genannten gespeicherten Daten eine Aufzeichnung zur Steuerung und/oder Überwachung darstellen.

11. Verfahren nach Anspruch 10, ferner umfassend die Abgabe eines Warnsignals, wenn der Inhaltsdetektor nach der Entnahme der biologischen Proben aus dem Behälter (1) weiterhin Inhalt erfasst, und/oder die Abgabe eines Warnsignals, wenn die Temperatur des Behälters (1) während des Transports einen zuvor festgelegten Temperaturschwellenwert erreicht oder überschreitet.

12. Verfahren nach Anspruch 10 oder 11, wobei das genannte Senden der Datenreihe in einem bestimmten einstellbaren Zeitintervall erfolgt.

13. Verfahren nach Anspruch 10, ferner umfassend das Verfolgen von Positionsdaten des Behälters (1) ab dem Zeitpunkt des Schließens des Behälters (1) bis zum Zeitpunkt des Öffnens des Behälters (1) während des Transports unter Einsatz einer mit dem Server (21) operativ verbundenen Positioniervorrichtung einer mobilen Rechnereinheit (20), wobei die genannte mobile Recheneinheit (20) mittels einer darin installierten mobilen Anwendung ferner am Ausgangsort zur Validierung der mit dem Behälter (1) verknüpften Ausgangsdaten bzw. des Ausgangsortes des RFID-Transponders (40) und am Zielort zur Validierung der Zieldaten bzw. des Zielortes des Behälters (1) und zur Aktivierung des RFID-Lesegeräts verwendet wird, wobei das System ferner eine Warneinheit zur Abgabe eines Alarms bei Auftreten eines Fehlers umfasst.

## Revendications

1. Un système pour le transport contrôlé d'échantillons biologiques comportant :
- un serveur (21) comprenant une base de données ; et
- un récipient (1) pour stocker les échantillons biologiques comprenant :
- un corps principal (3) définissant une cavité du boîtier ;
- un couvercle (2) ;
- un dispositif de contrôle d'ouverture/fermeture pour contrôler l'ouverture/fermeture du récipient (1);
- au moins un capteur de température configuré pour fournir des informations sur la température interne du récipient (1) depuis un moment de chargement de ces échantillons biologiques dans le récipient (1) jusqu'à un moment postérieur d'ouverture du récipient (1) et/ou d'extraction des échantillons biologiques du récipient (1) ; et
- une carte électronique configurée pour collecter des données acquises par cet au moins un capteur de température et pour contrôler un état et un fonctionnement de ce dispositif de contrôle d'ouverture/fermeture, où la carte électronique comprend un module de communication configuré pour envoyer à ce serveur (21), durant le transport, une série de données pour le stockage de cette série de données dans la base de données du serveur (21) ;
où :
- le système comprend par ailleurs deux tags RFID différents (40, 41), où un premier tag RFID (40) comprend des données d'un centre d'origine et un deuxième tag RFID (41) comprend des données d'un centre de destination du récipient (1) ainsi que le type d'échantillon biologique à transporter ;
- le récipient (1) comprend par ailleurs un lecteur RFID qui est configuré pour lire les données de ces différents tags RFID (40, 41) à un certain moment configuré ;
- le dispositif de contrôle d'ouverture/fermeture comporte un dispositif électromécanique qui est configuré pour être activé durant l'ouverture ou la fermeture, en utilisant les tags RFID (40, 41) ; et
- ce récipient (1) comprend par ailleurs un détecteur d'échantillons biologiques à l'intérieur du récipient (1) contrôlé par cette carte électronique,
où ce détecteur d'échantillons biologiques est un capteur de proximité sélectionné parmi les détecteurs infrarouge, capacitif ou inductif, le capteur de proximité étant configuré pour détecter la présence d'échantillons biologiques transportés à l'intérieur du récipient (1) depuis le moment d'y charger des échantillons biologiques jusqu'au moment d'extraire les échantillons biologiques du récipient (1); et
où la série de données envoyées par la carte électronique comporte des données sur le contenu du récipient (1), acquises par le détecteur et des données sur la température interne du récipient (1), de sorte que le système assure que toute manipulation du contenu du récipient (1) soit enregistrée.

2. Le système conformément à la revendication 1, où ces échantillons biologiques sont retenus à l'intérieur d'un récipient secondaire (14) aménagé à l'intérieur du récipient (1).

3. Le système conformément à la revendication 2, où le récipient secondaire (14) est fait en un matériau transparent ou translucide et comporte par ailleurs une étiquette, une plaque ou une zone texturisée (25) pour coopérer avec le capteur de proximité.

4. Le système conformément à une quelconque des revendications précédentes, où le dispositif de contrôle d'ouverture/fermeture est compris dans le couvercle (2).

5. Le système conformément à la revendication 1, comportant par ailleurs un dispositif informatique mobile (20) configuré pour la communication sans fils avec ce module de communication et avec le lecteur RFID, par le biais d'une application mobile qui y est installée, où ce dispositif informatique mobile (20) est configuré pour être utilisé, à l'origine. pour valider les données et le centre d'origine associé au récipient (1) et à la destination, pour valider les données et le centre de destination et pour activer le lecteur RFID, le système comprenant par ailleurs une unité d'avertissement pour émettre une alarme en cas d'erreur.

6. Le système conformément à une quelconque des revendications précédentes, comportant par ailleurs une unité d'affichage (5) contrôlée par la carte électronique pour indiquer des informations différentes sur le contenu du récipient (1) qui est au moins reliée aux échantillons biologiques transportés où cette unité d'affichage comporte un écran et/ou des indicateurs lumineux.

7. Le système conformément à une quelconque des revendications précédentes, où le récipient (1) comporte par ailleurs une ou plusieurs batteries pour l'autoalimentation de la carte électronique.

8. Le système conformément à une quelconque des revendications précédentes, où le récipient (1) comporte par ailleurs au moins un accéléromètre configuré pour détecter des mouvements soudains du récipient (1) durant le transport ou la manipulation.

9. Le système conformément à une quelconque des revendications précédentes, comportant par ailleurs un dispositif informatique (20), comprenant un téléphone portable ou une tablette, situé/e à proximité du récipient (1) durant le transport, ce dispositif informatique (20) comprenant des moyens de positionnement connectés fonctionnellement au serveur (21) pour permettre de suivre la trace des données de positionnement du récipient (1) durant le transport.

10. Une méthode pour le transport contrôlé d'échantillons biologiques dans un récipient, où ce récipient (1) comporte :
- un corps principal (3) définissant une cavité du boîtier ;
- un couvercle (2) ;
- un lecteur RFID ;
- un dispositif de contrôle d'ouverture/fermeture pour contrôler l'ouverture/fermeture du récipient (1) où le dispositif de contrôle d'ouverture/fermeture comporte un dispositif électromécanique configuré pour être activé, durant l'ouverture ou la fermeture, en utilisant au moins deux tags RFID différents (40, 41) ;
- au moins un capteur de température fournissant des informations sur la température interne du récipient (1) depuis un moment de chargement des échantillons biologiques dans le récipient (1) jusqu'à un moment postérieur d'ouverture du récipient (1) et/ou d'extraction des échantillons biologiques du récipient (1) ; et
une carte électronique configurée pour collecter les données acquises par au moins ce capteur de température et pour contrôler un état et le fonctionnement de ce dispositif de contrôle d'ouverture/fermeture ;
la méthode comportant les étapes suivantes :
la lecture par le lecteur RFID de données d'au moins deux tags RFID différents (40, 41) à un certain moment configuré, où un premier tag RFID (40) comprend des données d'un centre d'origine et un deuxième tag RFID (41) comprend des données d'un centre de destination du récipient (1) ainsi que le type d'échantillon biologique à transporter ;
la détection, au moyen d'un détecteur d'échantillons biologiques contrôlé par cette carte électronique, de l'existence d'un chargement d'échantillons biologiques dans le récipient (1) ;
où ce détecteur d'échantillons biologiques est un capteur de proximité sélectionné parmi les détecteurs infrarouge, capacitif ou inductif, le capteur de proximité étant configuré pour détecter la présence d'échantillons biologiques transportés à l'intérieur du récipient (1) depuis le moment d'y charger des échantillons biologiques jusqu'au moment d"extraire les échantillons biologiques du récipient (1).
l'envoi à un serveur (21), au moyen d'un module de communication compris dans la carte électronique, d'une série de données sur la température interne du récipient (1), le contenu et les incidences d'ouverture/fermeture du récipient (1) et/ou les incidences reliées à la manipulation du contenu ; et
le stockage de cette série de données reçues et des moments de ces incidences d'ouverture/fermeture du récipient (1) dans une base de données du serveur (21),
où ces données stockées dans cette base de données constituent un enregistrement de contrôle et/ou de supervision.

11. La méthode conformément à la revendication 10, comportant par ailleurs l'émission d'un signal si le détecteur de contenu continue de détecter le contenu après l'extraction des échantillons biologiques du récipient (1) et/ou l'émission d'un signal si la température du récipient (1) est la même ou si elle dépasse une valeur de température seuil préétablie durant le transport.

12. La méthode conformément aux revendications 10 ou 11, où cet envoi de la série de données est effectué chaque certain temps configurable.

13. La méthode conformément à la revendication 10, comportant par ailleurs, des données cherchant la trace de positionnement du récipient (1) depuis un moment de fermeture du récipient (1) jusqu'à un moment d'ouverture du récipient (1), durant le transport, en utilisant des moyens de positionnement d'un dispositif informatique mobile (20) fonctionnellement connecté au serveur (21), ce dispositif informatique mobile (20) étant par ailleurs utilisé au moyen d'une application mobile qui y est installée pour la validation à une origine des données du tag RFID (40) et centre d'origine associé au récipient (1) et à une destination pour valider les données du tag RFID (41) et le centre de destination du récipient (1) et pour activer le lecteur RFID, le système comprenant par ailleurs une unité d'avertissement pour émettre une alarme en cas d'erreur.
